## Europäisches Patentamt

## European Patent Office

## Office européen des brevets

(19)

(11) Publication number: **0 511 202 B1**

## EUROPEAN PATENT SPECIFICATION

(12)

(45) Date of publication of patent specification: **01.06.94**

(51) Int. Cl.5: **A61K 39/395**, C07K 15/28, C07K 15/06, C12N 5/16, C12P 21/08

(21) Application number: **90901787.3**

(22) Date of filing: **19.01.90**

(86) International application number: **PCT/EP90/00111**

(87) International publication number: **WO 91/10448 (25.07.91 91/17)**

(54) **A CELL SURFACE ANTIGEN ASSOCIATED WITH CELLULAR APOPTOSIS.**

(43) Date of publication of application: **04.11.92 Bulletin 92/45**

(45) Publication of the grant of the patent: **01.06.94 Bulletin 94/22**

(84) Designated Contracting States: **AT BE CH DE DK ES FR GB IT LI LU NL SE**

(56) References cited:

Science vol. 245. 1989. Trauth et al. pp 301-305

Dialogue Information Service. File 154. Medline 83-90/Oct. Takahashi et al.

Nature vol. 337. 1989 Smith et al.pp 181-184

Dialogue Information Service File 154. Medline 83-90/Oct. Acuto et al.

Dialogue Information Service. Medline 83-90/Oct. File 154. Schwarting et al.

Science vol. 248. 1990. Blackman et al. pp 1335-1341.

(73) Proprietor: **GERMAN CANCER RESEARCH CENTER**
**P.O. Box 10 19 49**
**W-6900 Heidelberg(DE)**

(72) Inventor: **KRAMMER, Peter, H.**
**Im Neuenheimer Feld 280**
**D-6900 Heidelberg(DE)**

(74) Representative: **Kraus, Walter, Dr. et al**
**Patentanwälte Kraus, Weisert & Partner**
**Thomas-Wimmer-Ring 15**
**D-80539 München (DE)**

**Description**

Cell surface molecules play crucial roles in lymphocyte growth control. Such molecules may function as receptors for growth-stimulating cytokines or be associated with receptors and transmit signals essential for growth regulation. Receptor blockade or removal of the stimulating cytokines can lead to decreased lymphocyte growth. For example, withdrawal of interleukins slows human lymphocyte growth and finally leads to a characteristic form of cell death called "programmed cell death" or apoptosis. E. Duvall and H. H. Wyllie, Immunol. Today 7, 115 (1986). Apoptosis is the most common form of eukaryotic cell death and occurs in embryogenesis, metamorphosis, tissue atrophy, and tumor regression. A. H. Wyllie, J. F. R. Kerr, A. R. Currie, Int. Rev. Cytol. 68, 251 (1980). It is also induced by cytotoxic T lymphocytes and natural killer and killer cells; by cytokines such as tumor necrosis factor (TNF) and lymphotoxin (LT); and by glucocorticoids. The characteristic signs of apoptosis are segmentation of the nucleus, condensation of the cytoplasm, membrane blebbing (zeiosis), and DNA fragmentation into multimers of about 180 base pairs (called a "DNA ladder").

Recently, it has been shown that anti-CD3 induces apoptosis of immature thymocytes in vitro. C. A. Smith et al., Nature 337, 181 (1989). It has been suggested that CD3-triggered apoptosis might be responsible for negative selection of T cells in the thymus.

The selective induction of apoptosis in cells, such as diseased cells, could prove a useful therapeutic tool.

Monoclonal Antibody-Mediated Tumor Regression by Induction of Apoptosis is described in Science, Vol. 245, July 1989, pp 301-305.

The present invention relates to the use of an antibody which specifically binds to an antigen associated with cellular apoptosis under conditions appropriate for antibody mediated cellular apoptosis for the preparation of a therapeutic composition for the inhibition of the proliferation of a cell infected by the HTLV-1 virus as well as to the use of an antibody which specifically binds to an antigen associated with cellular apoptosis under conditions appropriate for antibody mediated cellular apoptosis for the preparation of a therapeutic composition for killing a substantial percentage of a population of cells infected by the HTLV-1 virus.

APO-1 is associated with cellular apoptosis and has a molecular weight of about 52 kD and is expressed by activated and malignant lymphoid cells. The binding of antibody to APO-1 induces apoptosis and thus, antibody or analogous binding agents can be used to induce growth inhibition or apoptosis in cells, such as lymphoid tumor cells, which carry the APO-1 antigen.

Figure 1 shows an autoradiogram of a SDS polyacrylamide electrophoretic gel for determination of the molecular weight of APO-1.

Figure 2 shows the induction of growth inhibition and apoptosis by anti-APO-1.

Figure 3 illustrates anti-APO-1-induced regression of a lymphoma in mice.

Figure 4 shows the localization of anti-APO-1 antibody in xenografts of a lymphoid tumor.

The antigen APO-1 is a cellular membrane antigen having a molecular weight of appoximately 52kD as determined by SDS-polyacrylamide gel electrophoresis. APO-1 is expressed by activated normal human lymphoid cells and lymphoid tumor cells, including B cell, T cell and HTLV-1-associated malignant cells such as adult T cell leukemic cells. The binding of anti-APO-1 antibody to cells expressing APO-1 results in growth inhibition and/or apoptosis. This effect is complement independent, mediated by antibody alone.

APO-1 can be isolated from the cellular membrane of cells (such as lymphoid cells) which express the antigen by conventional techniques. Further, the gene encoding APO-1 can be cloned and expressed to provide the isolated antigen or portions of it. Isolated APO-1 can be used as immunogen to raise anti-APO-1 antibody (polyclonal or monoclonal) or to screen for production of anti-APO-1 antibody by hybridomas, of chimeric anti-APO-1 antibody by transfected myelomas or of single chain anti-APO-1 antibody by transformed bacterial cells.

Antibodies which bind to APO-1 are useful for inducing inhibition of cell growth or apoptosis in cells that express APO-1. For this purpose, monoclonal anti-APO-1 antibodies are preferred. Monoclonal anti-APO-1 antibodies are produced by continuous (immortalized), stable, antibody-producing cell lines. The preferred antibody-producing cell lines are hybridoma cell lines. In principle, however, the cell lines can be derived from any cells which contain and are capable of expressing functionally rearranged genes which encode variable regions of the light and/or heavy chains of anti-APO-1 specificity. Preferably, the cell line should have the capability to assemble the chain (in the case of a single chain antibody) or chains into functional antibodies or antibody fragments. Thus, lymphoid cells which naturally produce immunoglobulin are preferred.

Hybridoma cells which produce monoclonal anti-APO-1 antibodies can be made by the standard somatic cell hybridization procedure of Kohler and Milstein, Nature 256: 495 (1975). Briefly, a procedure is as follows: the monoclonal anti-APO-1 antibodies are produced by immunizing an animal with whole cells bearing APO-1 or membranes of these cells. Alternatively, the animals can be immunized with purified or partially purified APO-1 or peptidic segments carrying one or more immunogenic epitopes of APO-1. Such peptides can be synthesized and conjugated to a carrier protein, such as keyhole limpet hemocyanin, to be used as an immunogen.

The preferred animal for immunization is the mouse. Various immunization protocols can be used. For example, mice can be given about 107 APA-1-bearing cells one per weak over a four-week period by intraperitoneal injection.

Antibody-producing lymphoid cells (e.g. splenic lymphocytes) are then obtained from the immunized animal and fused with immortalizing cells (preferably a myeloma or heteromyeloma). Many suitable myeloma cell lines are known in the art. For production of murine hybridomas, an example is the myeloma P3. X63.Ag8.653. See Kohler and Milstein, supra. Fusion of the spleen cells and fusion partner can be carried out in the presence of polyethylene glycol according to established methods. Techniques of electrofusion may also be used.

The resulting hybrid cells are clonally cultured and then screened for production of anti-APO-1 antibody. Hybridomas can be screened for secretion of antibodies which induce apoptosis against a cell line which expresses the APO-1 antigen. An example of such a cell line is the malignant human B cell line SKW6.4. Additional cell lines are set forth in table 1 below. Purified or partially purified APO-1 can be used to screen for hybridomas that secrete antibodies of APO-1 specificity by standard immunoadsorbant assays.

Although animal antibodies can be useful for human therapy, it may be preferable to convert animal antibodies to a form which may be better tolerated by a human. Monoclonal antibodies produced in murine or other animal systems can be converted to chimeric animal/human antibodies or to "near human" antibodies by standard techniques.

APO-1-binding fragments of analogues of anti-APO-1 antibodies can also be produced. For example, antibody fragments such as $F(ab')_2$, Fab and $F_V$ can be produced by enzyme digestion. In addition, synthetic oligopeptides representing Fab and $F_V$ analogues (single chain antibodies) can be produced in bacterial cells by genetic engineering techniques.

The antibodies can be used to induce growth inhibition or apoptosis in lymphoid cells (normal or malignant) or other cells bearing APO-1. For example, anti-APO-1 antibody can be used to treat tumors bearing the APO-1 antigen. As mentioned above, growth inhibition and/or apoptosis can be induced by antibody in various types of lymphoid cell malignancies which express APO-1. These lymphoid cell malignancies include malignancies of B or T cell lymphocytes. In particular, adult T cell leukemia, an HTLV-1 associated tumor, can be treated with anti-APO-1 antibody. In addition nonlymphoid tumors which bear APO-1 are candidates for the antibody therapy.

The anti-APO-1 antibodies are administered to a patient afflicted with the tumor in an amount that induces growth inhibition or apoptosis of APO-1 bearing cells. Effective anti-tumor dosages and dosage regimens can be determined for the various types of tumors. Generally, the antibodies can be given intravenously in a pharmaceutical vehicle such as saline.

Lymphoid tumors which express APO-1 can also be treated extracorporeally. Blood cells or blood leukocytes are removed from the patient and contacted with anti-APO-1 antibodies in amounts sufficient to reduce or eliminate tumor cells. After treatment, the blood cells or leukocytes are returned to the patient.

The invention is illustrated further by the following examples.

EXAMPLES

Example 1

Methods

Production of Anti-APO-1 Antibodies

BALB/c mice were immunized once per week over a 4-week period by intraperitoneal injection of 1 x $10^7$ SKW6.4 cells. Four days after the last injection, spleen cells from immunized animals were fused with the P3.X63.Ag8.653 myeloma [G. Kohler and C. Milstein, Nature 256, 495 (1975)]. Twelve days after fusion culture supernatants from wells positive for growth of SKW6.4 cells were tested for their ability to inhibit growth of SKW6.4 cells. Hybridomas that produced blocking monoclonal antibodies (MAbs) were cloned three times by limiting dilution at a concentration of 0.5 cells per well. MAbs were purified from serum-free culture supernatant by means of a protein A-Diasorb column (Diagen, Düsseldorf, FRG). Bound MAbs were eluted with 0.1M NaCl and 0.1 M glycine, pH 2.8, dialyzed against phosphate-buffered saline and sterilized. The isotype of the MAbs was determined by enzyme-linked immunosorbent assay [S. Kiesel, et al., Leuk. Res. 11, 1119 (1987)] with isotype-specific goat anti-mouse Ig that had been conjugated with horse radish peroxidase (Dunn, Asbach, FRG).

Determination of binding affinity and number of binding sites

Affinity and number of anti-APO-1 binding sites per cell were determined by Scatchard analysis as described [I. von Hoegen, W. Falk, G. Kojouharoff, P. H. Krammer, Eur. J. Immunol. 19, 239 (1989)]. Briefly, MAbs were iodinated by the IODO-gen method [P. J. Fraken and J. C. Speck, Biochem. Biophys. Res. Commun. 80, 849 (1980]. Aliquots of $5 \times 10^6$ cells were resuspended in 200 $\mu$l of culture medium containing 0.1% $NaN_3$, and different concentrations of $^{125}$I-labelled MAbs. After incubation at 4°C for 4 hours, two 95-$\mu$l portions were removed and centrifuged as described above by von Hoegen et al.

SDS polyacrylamide gel electrophoresis (SDS-PAGE) for molecular weight determination

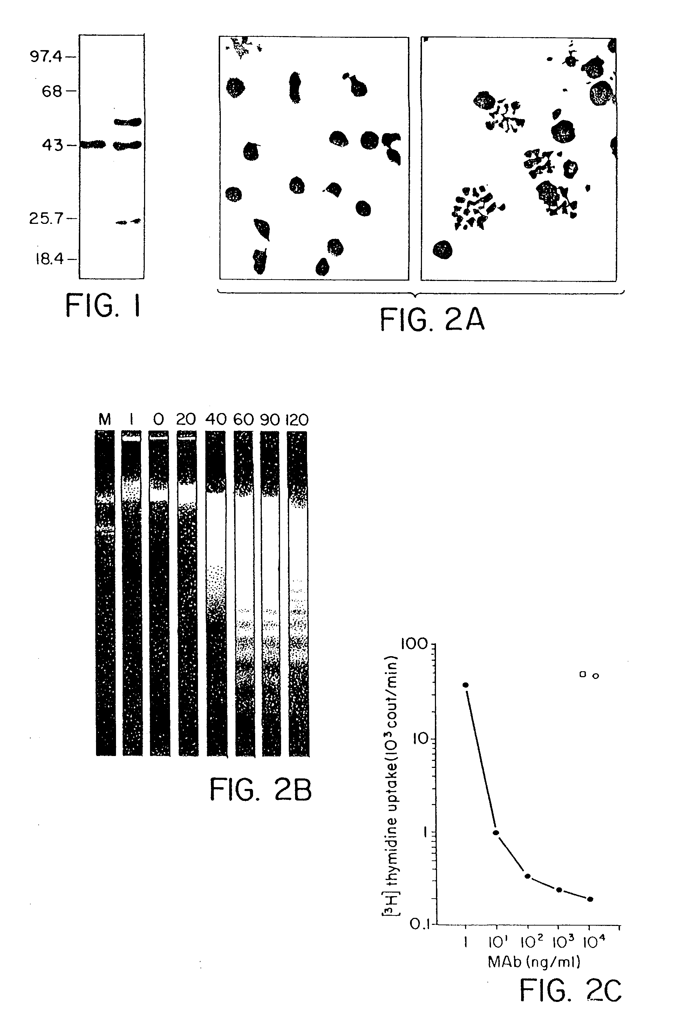

Cells ($3 \times 10^6$) were labelled with 60 $\mu$Ci of $^{75}$Se-labelledmethionine (Amersham, Braunschweig, FRG) in 6 ml of methionine-free culture medium (Biochrom, Berlin) for 48 hours. After washing the cells were incubated in either control MAb or anti-APO-1 (1 $\mu$g/ml) at 4°C for 45 min. The cells were washed and resuspended in lysis buffer (tris-buffered saline, pH 7.3, 1% Nonidet P-40, 1 mM phenylmethylsulfonyl fluoride, 0.1% aprotinin) at room temperature for 30 min. The lysates were centrifuged and supernatants were incubated with protein A-Sepharose beads (Pharmacia, Uppsala, Sweden) at 4°C for 1 hour. The immune complexes were washed four times with buffer (tris-buffered saline, pH 7.3, 0.25% Nonidet P-40) and resuspended in SDS-PAGE sample buffer containing 5% SDS and 5% 2-mercaptoethanol. The samples were heated to 95°C, centrifuged, and counts per minute of the supernatants were determined in a $\gamma$-counter. A total of 15,000 cpm were loaded in each lane and analyzed by a 10% SDS-PAGE (V. K. Laemmli, Nature 277, 680 (1970)). The gel was dried and subjected to autoradiography. The autoradiograph is shown in Figure 1; bands represent the immunoprecipitation of the biosynthetically labelled APO-1 with either the isotype-matched control MAb (left lane) or anti-APO-1 (right lane). The numbers on the left margin indicate the positions of the size markers.

Induction of growth inhibition and apoptosis by anti-APO-1

(A) The T cell line CCRF-CEM.S2 was cultured in the presence of purified MAb (1 $\mu$g/ml) in a microtiter plate for 2 hours before photography (left panel of Figure 2, control MAb 13BI; right panel, anti-APO-1). The CCRF-CEM.S2 subclone was obtained by cloning cells under limiting dilution conditions from the CCRF-CEM.S2 T cell line at one cell per well in 96-well microtiter plates. CCRF-CEM.S2 was selected because of its high sensitivity to programmed cell death induced by APO-1 (500 ng/ml) as measured by microscopic inspection in a 4-hour culture.

(B) CCRF-CEM.S2 cells ($10^6$ per milliliter) were incubated with MAb (1 $\mu$g/ml) in culture medium at 37°C. At various times, aliquots of $10^6$ cells were removed and DNA was prepared. In Figure 2B, M, marker; I, control MAb 13BI for 2 hours; lanes 3 to 7, anti-APO-1 for the times indicated). (C) SKW6.4 cells were either incubated with the isotype matched control MAb FII20 (□), FII23 (non-binding MAb) (O), or anti-APO-1 (●) in microcultures for 24 hours before labelling with [$^3$H] thymidine for a further 4 hours. The data represent the mean of duplicate cultures with a variation of less than 5%. The cells were cultured in RPMI 1640 medium (Gibco, Grand Island, New York), supplemented with 2 mM L-glutamine, streptomycin (100 $\mu$g/ml), penicillin (100 U/ml), 20 mM Hepes buffer pH 7.3, and 10% heat-inactivated fetal bovine serum (Conco Lab-Division, Wiesbaden, FRG). For microcultures $1 \times 10^4$ per well were cultured in duplicates in flat bottom 96-well microtiter plates (Tecnomara, Fernwald, FRG) (200 $\mu$l final volume per well). After 24 hours, the cells were labelled with 0.5 $\mu$Ci of [$^3$H]thymidine (Amersham, Braunschweig, FRG) for 4 hours. Before harvesting, the microcultures were examined by microscopic inspection. DNA fragmentation $1 \times 10^8$ cells were washed with cold phosphate-buffered saline and disrupted with NTE buffer, pH 8 (100 mM NaCl, 10 mM tris, 1 mM EDTA) containing 1% SDS and proteinase K (0.2 mg/ml). After incubation for 24 hours at 37°C, samples were extracted twice with phenol plus chloroform (1:1, v/v) and precipitated by ethanol. The DNA was dissolved in 38 $\mu$l of NTE

buffer and digested with ribonuclease (1 mg/ml) for 30 min at 37°C. To each sample 10 $\mu$l of loading buffer containing 15% Ficoll 400 (Pharmacia, Uppsala, Sweden), 0.5% SDS, 50 mM EDTA, 0.05% bromophenol blue, 0.05% xylene cyanol in TBE buffer (2 mM EDTA, 89 mM boric acid, 89 mM tris, pH 8.4) were added. The mixture was loaded onto a 1% agarose gel and stained after electrophoresis with ethidium bromide (0.5 $\mu$g/ml). The size marker was Hind III + ECO RI-digested $\lambda$ DNA.

Reactivity of anti-APO-1 with different cells

Aliquots of $10^8$ cells were incubated at 4°C in 100 $\mu$l of medium with control MAb (FII23 or I3BI) or anti-APO-1 for 30 min. Then the cells were washed and stained with fluorescein isothiocyanate-coupled goat anti-mouse IgF(ab')$_2$(70 $\mu$g/ml) and analyzed by a cytofluorograph (Ortho Diagnostic Systems, Westwood, Massachusetts).

For determination of the effect of anti-APO-1 on tritiated thymidine uptake, cells ($10^4$ per well) were cultured in the presence of MAb (500 ng/ml) for 24 hours and labelled with [$^3$H]thymidine for 2 hours before harvest; the data in table 1 represent the mean of duplicate cultures with a variation of less than 5%.

Leukemic cells from patients were obtained as follows. Bone marrow cells isolated from the patients were morphologically >95% blasts and showed the following phenotype: pre T-ALL, cytoplasmic CD3$^+$, CD5$^+$, CD7$^+$, CD34$^+$, Tdt$^+$, CD2$^-$, surface CD3$^-$, CD4$^-$, and CD8$^-$; T-ALL, CD2$^+$, cytoplasmic CD3$^+$, CD5$^+$,CD7$^+$ and Tdt$^+$, surface CD3$^-$, CD4$^-$, CD8$^-$ and CD34$^-$; common-ALL CD10$^+$, CD19$^+$, CD22$^+$, CD24$^+$, CD20$^-$ . The effect of anti-APO-1 on these leukemic cells was not tested, because they died under normal culture conditions.

Normal human lymphocytes were obtained as follows. Peripheral blood mononuclear cells (PBMC) from healthy volunteers were isolated by Ficoll Paque (Pharmacia Inc., Uppsala, Sweden) density centrifugation.Adherent cells were removed by adherence to plastic culture vessels overnight. T cells were isolated from PBMC by rosetting with 2 amino-ethylisothyouronium-bromide (AET)-treated sheep red blood cells as described (20). Freshly prepared resting T cells (2 x $10^6$ per milliliter; 96% OKTII$^+$, 1% Tac$^+$) were activated with phytohemagglutinin-M (50 $\mu$g/ml)) and PMA (10 ng/ml) (Sigma Chemical Co., Munich, FRG). Two, 7 and 12 days later the T cells were fed with 20 to 30 U/ml or recombinant human interleukin-2 (20 to 30 U/ml). T cells (5 x $10^5$ per milliliter) activated for 12 days (90% OKTII$^+$; 60% Tac$^+$) were cultured in the presence of FII23 or anti-APO-1 (1 $\mu$g/ml) in triplicates for 24 hours and then labelled with [$^3$H]thymidine for a further 17 hours (see above).

Resting B cells (35.8% CD19$^+$) were isolated by two rounds of rosetting as above, followed by separation via a Sephadex® G-10 column as described. T.R. Jerrells, J.H. Dean, G.L. Richardson, D.B.Herberman, J. Immunol. Methods 32,11 (1980). For activated B cells, PBMC were adjusted to 2 x $10^6$ cells per milliliter and cultured in the presence of pokeweed mitogen at 10 $\mu$g/ml (Serva, Heidelberg, FRG) for 6 days. Dead cells and T cells were then eliminated by rosetting with AET-treated sheep red blood cells and subsequent centrifugation over Ficoll Pague. The interphase cells were used as activated B cells (84% sIgM$^+$).

Effect of anti-APO-1 on tumor growth in vivo

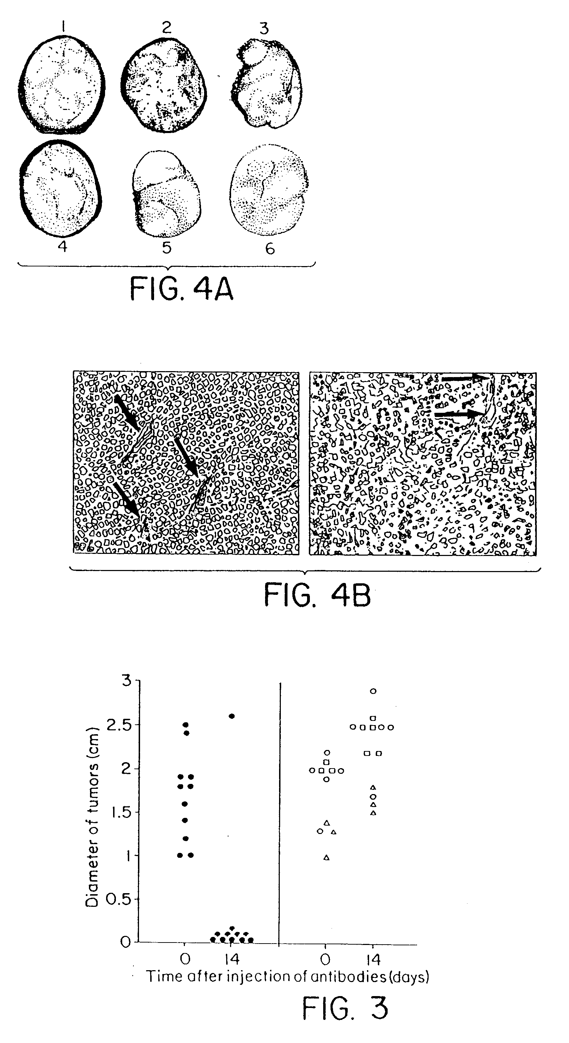

BJAB cells (4 x $10^7$) were injected subcutaneously into the left flank of $\eta\mu/\eta\mu$ mice. After 5 weeks (day 0) the mice were injected with 500 $\mu$g of MAb into the tail vein. The results are shown graphically in Figure 3. Control MAb FII20 (□); FII23 (O); I3BI (△); and anti-APO-1 (●). Fourteen days later the size of the tumors was measured at the base of the tumor; the tumors from individual mice are represented by dots.

Localization of anti-APO-1 in tumor xenografts

Localization of anti-APO-1 in xenografts of BJAB in $\eta\mu/\eta\mu$ mice and induction of apoptosis of the tumor is illustrated in Figure 4. Figure 1A, upper row, shows the uptake of $^{125}$I-labelled MAb anti-APO-1 (50 $\mu$g, 50 $\mu$Ci per mouse) in the tumor at 12 hours (1), 48 hours (2), and 96 hours (3) after intravenous injection of the MAb. The lower row shows the uptake of $^{125}$I-labelled MAb anti-APO-1 500 $\mu$g, as used for therapy; 50 $\mu$Ci per mouse) (4) and FII20 (control MAb, binding to BJAB; 50 $\mu$g, 50 $\mu$Ci per mouse) (5) and $^{125}$I-labelled FII23 (control MAb, nonbinding to BJAB; 50 $\mu$g, 50 $\mu$Ci per mouse) (6) in 48 hours, respectively.

Ten days after intravenous injection of 500 $\mu$g of MAb per mouse the remaining tumor tissue was removed and fixed with formalin. Paraffin sections of the tumor were stained with haematoxylin/eosin.

In Figure 4B, the left panel shows tumor after treatment with control MAb FII20; the right panel shows tumor after treatment with anti-APO-1. Arrows indicate host vessels. Final magnification, x 92. MAbs were radioiodinated according to the IODO-Gen method (see above). Labelled MAbs were injected into the tail vein and animals were killed by ether anesthesia at the predetermined time points. The tumors were excised and embedded in methyl-cellulose and 20-$\mu$m cryotome sections were prepared. Lyophilized sections were placed on a Kodak X-omat AR film for autoradiography.

## Results and discussion

A MAb (anti-APO-1) was identified that blocks growth and induces apoptosis of SKW6.4 cells. The anti-APO-1 (IgGG3, $\chi$, $K_D$ = 1.9 x $10^{-10}$ ) bound to approximately 4 x $10^4$ sites on the surface of SKW6.4 cells. The antibody specifically immunoprecipitated an endogenously synthesized protein antigen (APO-1) from SKW6.4 cells which, under reducing conditions, was observed on SDS-polyacrylamid gel electrophoresis (SDS-PAGE) as a main band of 52 kD (Figure 1). Apart from actin (43 kD), which was nonspecifically precipitated with IgG3, anti-APO-1 specifically immunoprecipitated a minor band of 25 kD. This 25-kD protein might either represent a degradation product or be noncovalently associated with the 52-kD protein.

There are two major modes of death in nucleated eukaryotic cells. Necrosis as a result, for example, of complement attack is characterized by swelling of the cells and rupture of the plasma membrane caused by an increase in permeability. Cells that undergo apoptosis, however, show a different biochemical and morphological pattern. This pattern corresponds to the one induced by anti-APO-1: condensation of the cytoplasm, membrane blebbing (Figure 2a), and endonuclease-induced DNA fragmentation (A.H. Wyllie, Nature 284, 555 (1980)) into multimers of approximately 180 bp (Figure 2b). Affinity-purified anti-APO-1 induced growth retardation and cell death (Figure 2c), which was not observed with either an isotype-matched, control MAb (FII20) [anti-MHC(major histocompatability complex) class I antigens] or the nonbinding MAb FII23. Abrogation of [$^3$H]thymidine incorporation along with increased trypan blue uptake into dead cells were observed, and growth of $10^4$ SKW6.4 cells in 200-$\mu$l cultures was blocked by more than 95% by an anti-APO-1 concentration of only 10 ng/ml (Figure 2c). The specificity of cell death induced by anti-APO-1 becomes evident from the fact that the following additional control MAbs were inactive for induction of apoptosis: 18 nonbinding and 9 binding MAbs of the IgG3 isotype (tested by immunofluorescence on SKW6.4 cells) and a panel of MAbs directed against known antigens on the cell surface of SKW6.4 cells including CD19, CD20, CD22, MHC class II, IgM (immunoglobulin M), and the SKW6.4 idiotype. (Monoclonal anti-CD19 (HD37) and anti-CD22 (HD39) were kindly provided by B. Dorken (Polyclinic of the University, Heidelberg,, FRG) and monoclonal anti-CD20 by G. Moldenhauer (IV Leukocyte typing workshop and conference, Vienna, Austria, 1989), respectively. The 18 nonbinding and 9 binding MAbs of the IgG3 isotype (tested by immunofluorescence on SKW6.4 cells) and the MAbs directed against MHC class II, IgM, and SKW6.4Ig idiotypes were raised in our own laboratory).

Cell death induced by anti-APO-1 was complement-independent and occured under serum-free culture conditions or in culture medium plus serum inactivated at 56°C for 30 min. It differed from death mediated by complement-dependent lysis by: (i) morphology and formation of a DNA ladder (Figure 2, a and b), (ii) exogenous $Ca^{2+}$ independence and (iii) delayed $^{51}$Cr release from radiolabelled target cells. The kinetics of membrane blebbing induced by anti-APO-1 (within 30 min; Figure 2a) was not influenced by the presence of 10mM EDTA or EGTA. In addition, endonuclease-mediated DNA fragmentation induced by anti-APO-1 was not inhibited by the $Ca^{2+}$ channel blockers Furamicin (50 $\mu$M) or Nifedipin (50 $\mu$M). When $^{51}$Cr-labelled SKW6.4 cells were incubated with anti-APO-1 (1 $\mu$g/ml) for 2, 4, 8 and 24 hours, the specific $^{51}$Cr release [R.C. Duke, R. Chervenak, J.J. Cohen, Proc. Natl. Acad. Sci. U.S.A. 80, 6361 (1983)] was found to be 2.9%, 7.6%, 21.3% and 32.5%, respectively. Trypan blue uptake was measured at the same time points: 2.5%, 4.7%, 10.6% and 73.6%, respectively, of the cells were trypan blue positive. In contrast, 2 hours after the addition of MAbs, plus complement the specific $^{51}$Cr release was 108.7% and 92.7% of the cells stained with trypan blue. These experiments indicate that cell death induced by anti-APO-1 is fundamentally different from antibody- and complement-dependent cell lysis.

To assess the specificity of anti-APO-1, a restricted panel of tumor cell lines was screened for expression of APO-1 and susceptibility to growth inhibition and apoptosis. APO-1 was expressed on various human lymphoid B and T cell lines and was not found on a gibbon or mouse T cell line or a human monocytic cell line (Table 1). Anti-APO-1 blocked proliferation of the APO-1-positive cell lines listed in Table 1 via induction of apoptosis, and formation of a DNA ladder was observed in each case. Two hours after addition of the MAbs (1 $\mu$g/ml) the genomic DNA of each tumor line was isolated and analyzed on agarose gels as described above. Inhibition of [$^3$H]thymidine uptake by anti-APO-1 was paralleled by fragmentation of the genomic DNA. This was not observed after treatment with control MAb (I3BI).

Expression of APO-1 was not restricted to cell lines in vitro but could be found on leukemic cells freshly isolated from patients (Table 1). Since APO-1 was not found on all leukemic cells anti-APO-1 may define a subpopulation of leukemias.

We also screened human B and T cells for expression of APO-1. We did not detect APO-1 on resting B cells. However, APO-1 was expressed on activated B cells (Table 1) and IgM secretion was reduced approximately fourfold by 3 days of treatment with anti-APO-1. (Activated B cells ($10^6$ per milliliter) were incubated in the presence of MAb FII23 or anti-APO-1 at 1 ug/ml. After 3 days the culture supernatants were collected and the IgM concentration measured with a human IgM-specific ELISA containing HRPO-conjugated goat anti-human IgM (Medac, Hamburg, FRG). IgM secretion after treatment with FII23 or anti-APO-1 was 2100 and 550 ng/ml, respectively).

Table 1. Reactivity of anti-APO-1 with different cells

| Cells* | | Cells positive for APO-1 (%) | Relative fluorescence intensity (anti-APO-1/control) | Effects of MAbs on [³H]thymidine uptake (10³ cpm) | |
|---|---|---|---|---|---|
| Type | Designation | | | Control | Anti-APO-1 |
| **Malignant cell lines** | | | | | |
| Hu B cells | SKW6.4 | 98 | 11.1 | 30.0 | 0.02 |
| | CESS | 95 | 12.0 | 23.0 | 0.1 |
| | BJAB | 80 | 2.1 | 70.0 | 7.0 |
| | OCI.LY1 | 0 | 1 | 14.5 | 15.8 |
| Hu T cells | Jurkat | 83 | 2.3 | 20.3 | 8.1 |
| | Molt | 91 | 2.4 | 35.7 | 0.6 |
| | CCRF.GEM | 64 | 1.9 | 16.2 | 0.5 |
| Hu myeloid cells | U937 | 5 | 0.97 % | 62.2 | 60.5 |
| Gibbon T cells | MLA 144 | 0 | 0.96 | 34.3 | 35.0 |
| Mouse T cells | EL4 | 0 | 1 | 44.8 | 45.3 |
| **Leukemic cells from patients** | | | | | |
| Pre T-ALL | B.M. | 54 | 4.4 | | |
| T-ALL | D.A. | 53 | 3.2 | | |
| Common ALL | W.N. | 72 | 5.0 | | |
| **Normal human lymphocytes** | | | | | |
| T cells | Resting | 3 | 1.36 | | |
| | Activated | 89 | 7.4 | 22.4 | 0.23 |
| B cells | Resting | 0 | 0.9 | | |
| | Activated | 91 | 1.1 | | |

* Hu, human; ALL, acute lymphocyte leukemia.

Peripheral resting T cells did not express APO-1. Activated T cells, however, expressed APO-1 and anti-APO-1 induced apoptosis and growth inhibition of these cells (Table 1). Thus, our data suggest that APO-1 is a species-specific antigen expressed on activated or malignant lymphocytes.

The striking effect of anti-APO-1 in vitro prompted us to test its effect on tumor growth in vivo. Although the Epstein-Barr virus (EBV)-negative, Burkitt-like lymphoma BJAB was the least sensitive to anti-APO-1 of the B cell panel in Table 1 and expressed only approximately 1.5 x 10⁴ APO-1 epitopes per cell (4), we selected BJAB for our in vivo experiments. The reason for this choice was that only BJAB grew to large tumor masses in unirradiated $\eta\mu/\eta\mu$ mice. Five weeks after injection of BJAB cells the $\eta\mu/\eta\mu$ mice carried tumors with a diameter of appoximately 1.0 to 2.5 cm (Figure 3). These mice were injected intravenously with purified anti-APO-1 (500 $\mu$g per mouse) or the same quantities of various isotype-matched control antibodies (FII20, anti-MHC class I antigens, recognizing $5.8 \times 10^5$ sites per cell; or one of the two nonbinding MAbs FII23 and I3BI). As a control we also injected anti-APO-1 (500 $\mu$g per mouse) into three $\eta\mu/\eta\mu$ mice carrying the APO-1-negative B cell tumor OCI.LYI with tumor diameters of 1.5, 1.8, and 3.4 cm, respectively (OCI.LYI was obtained from H. Messner, Ontario Cancer Institute, Toronto, Canada) (see also Table 1). Two days after anti-APO-1 injection, a whitish discoloration of the BJAB tumors was observed that was followed by rapid tumor regression. Macroscopic tumor regression was seen in 10 of 11 treated mice within less than 14 days. The control antibodies had no effect (Figure 3). In addition, no tumor regression was observed in the mice carrying OCI.LYI, as expected. In addition, no tumor regression was observed in the mice carrying OCI.LYI, as expected.

To demonstrate proper localization and enrichment of the injected antibodies, labelled MAbs were visualized by autoradiography of sections of the BJAB tumor tissue (Figure 4a). These autoradiographs showed a pronounced binding of anti-APO-1 in the periphery but only sparse accumulation in the center of the tumor. The binding control MAb FII20 showed a qualitatively similar binding pattern. There was no localization of the nonbinding control MAb FII23 above background. Furthermore, paired label experiments (D. Pressman at al., Cancer Res. 17, 845 (1957)) with labelled anti-APO-1 and FII23 revealed that the specific enrichment of anti-APO-1 over FII23 in the tumor was four- and sixfold after 48 and 96 hours, respectively.

The main purpose of our experiments was to assess whether anti-APO-1 can also act in vivo. Therefore, the tumor-bearing mice only received one intravenous injection of anti-APO-1 at a dose in the range used in MAb therapies. In other therapy schedules, however, MAbs are injected repeatedly (see e.g. S.L. Brown et al., Blood 73, 651 (1989). In our experiments regrowth of the BJAB tumor was observed in three of the ten mice in which tumor regression had been observed (Figure 3). Regrowth was observed at the margin of the original tumor approximately 3 months after the initial macroscopic tumor regression. One of these tumors was removed and found to express APO-1 by immunofluorescence and to be sensitive to anti-APO-1 in vitro at a MAb concentration similar to the original in vitro BJAB tumor cell line (Table 1).

To determine the histology of the regressing BJAB tumors we prepared thin sections of tumors from MAb-treated $\eta\mu/\eta\mu$ mice. Ten days after intravenous injection of FII20, BJAB appeared as a solid tumor composed of densely packed large blasts with numerous mitoses, some tumor giant cells, and rare apoptotic figures (Figure 4b, left panel). The tumor was penetrated by host vessels. In contrast, almost all remaining BJAB cells of mice treated with anti-APO-1 (Figure 4b, right panel) showed severe cytopathic changes including nuclear pycnosis and cellular enema most pronounced in perivascular microareas. These morphological changes are characteristic of apoptosis.

Taken together, these data strongly suggest that apoptosis is induced by anti-APO-1 and is the mechanism of death and regression of BJAB tumor cells in vivo. In fact that FII20, which strongly binds to the cell surface of BJAB tumor cells, did not cause regression of BJAB also precludes the possibility that killer cells or complement that might have bound to anti-APO-1 may have been involved in the growth inhibition and tumor regression.

Example 2

Materials and Methods

Cell culture medium and reagents:

All cells were cultured in RPMI 1640 supplemented with L-glutamine (2 mM final concentration), streptomycin (100 $\mu$g/ml), penicillin (100 U/ml), HEPES (25 mM final concentration) and 10% fetal calf serum (FCS, Advanced Biotechnologies Inc., Silver Spring, MD). Recombinant IL-2 (final concentration 20 U/ml) and recombinant IL-4 (final concentration of 5 ng/ml) were purchased from Boeringer Mannheim, FRG. The cultures were kept at 37°C in 95% air, 5% $CO_2$ at 90% relative humidity.

Cell lines and leukemic cells from ATL patients:

Various HTLV-1 transformed T cell lines were investigated. C91/PI is a cord blood T cell line transformed by HTLV-I and continuously kept in culture in the presence of IL-2. All other T cell lines used were originally derived from patients with ATL:JGCL and DCL are IL-2 dependent cell lines. MJCL and MTI are IL-2 independent cell lines which still respond to IL-2 with enhanced proliferation. CRII2 and HUT102 are the prototype HTLV-I positive leukemic cell lines in which HTLV-1 was originally described. Growth of these cell lines does not depend on IL-2 in the culture medium.

As a prototype APO-1 positive and anti-APO-1 sensitive cell line, B cell line SKW6.4 was included as a control in all experiments. Trauth B.C., Klas C., Peters A.M.J., Matzku S., Moller P., Falk W., Debatin K-M, Drammer PH. "Monoclonal antibody-induced tumor regression by activation of an endogenous suicide programme." Science 1989; 245: 301-305.

Thawed leukemic cells from five patients with ATL were used for the experiments. The cells from patients with a high leukemic cell count were frozen and stored in liquid nitrogen in medium containing 20% FCS and 10% DMSO. After careful thawing there was a considerable loss of cells which appears to be characteristic for ATL cells. Viable cells were isolated after thawing by density gradient centrifugation (LSM, Organon Technika Corp., Durham, N.C.) and cultured in vitro for further experiments.

Immunofluorescence analysis

Immunofluorescence staining was determined by flow cytometry with the following antibodies: anti-Tac-antibody was used at 1 $\mu$g/10$^6$ cells. Anti-APO-1 (IgG3, ) and an isotype matched control non-binding antibody were used as a 10% dilution of the original hybridoma supernatant. Antibodies against CD3, CD4, CD8 were purchased from Becton Dickinson (Mountain View, CA.) and used according to the manufacturer's instructions. For cell surface staining 1 x 10$^6$ cells in 100 $\mu$l medium were incubated with the appropriate dilutions of the antibodies in phosphate buffered saline (PBS) containing 1% FCS and 0.3% Na-azide. Prior to the addition of antibodies human IgG was added to a final concentration of 100 $\mu$g/ml. After incubation for 30 m, cells were washed and incubated with a second FITC labelled goat-anti-mouse Ig antibody (TAGO Burlingame, Ca.).

Cell proliferation

Cell proliferation at the cell concentrations indicated was determined in 96 well flat bottom plates (Costar, Cambridge, Mass.). After the time indicated 0.1 $\mu$Ci of [$^3$H]thymidine ($^3$H-TdR, Dupont NEN, Boston, Mass.) was added to each culture well. Proliferation was assessed by harvesting the plates and $^3$H-TdR uptake was determined in a liquid scintillation counter.

Cell death

After incubation of cells with the anti-APO-1 antibody viability and cell death were determined by the trypan blue dye exclusion method.

Results

APO-1 expression on HTLV-I positive T cell lines

We first investigated the expression of the APO-1 antigen on various T cell lines originally established from patients with ATL. By immunofluorescence staining all cell lines used displayed a characteristic mature T cell phenotype (CD4$^+$, CD8$^-$, Tac$^+$). Only the JGCL cell line was CD4$^-$, CD8$^+$, Tac$^+$. Strong expression of the APO-1 antigen was found on all cell lines. The intensity of APO-1 expression was comparable to the expression found on activated normal T cells or on APO-1 positive B and T cell lines.

Inhibition of proliferation and anti-APO-1 induced apoptosis of HTLV-I positive T cell lines

To assess growth inhibition and apoptosis of HTLV-I positive T cell lines by anti-APO-1 the cell lines were cultured in vitro for two days in the presence of various concentrations of anti-APO-1. As a control parallel cultures were incubated with 10 $\mu$g/ml of an isotype-matched nonbinding control antibody. The proliferation of all T cell lines tested was inhibited by anti-APO-1 antibody. As a positive control, SKW6.4, the highly anti-APO-1 sensitive cell line, a B lymphoblastoid line against which anti-APO-1 was originally raised (see example 1) was includes in each experiment. Growth inhibition of various cell lines, e.g. JGCL and MJCL, was quantitatively comparable to growth inhibition of the SKW6.4 cell line.

All highly sensitive cell lines showed the characteristic morphological features of apoptosis (membrane blebbing, condensation of nucleus and cytoplasm) after incubation with anti-APO-1. After two days of incubation with anti-APO-1 50-98% of the cells were found to be dead by the trypan blue dye exclusion method.

## Expression of APO-1 on cultured ATL cells

After we had observed APO-1 expression and induction of apoptosis by anti-APO-1 on the HTLV-1 positive T cell lines, we tested whether APO-1 is also expressed on the leukemic cells from patients with ATL. Thawed cells from frozen peripheral blood cells (more than 50% malignant T cells) were investigated. The recovery of ATL cells after thawing is usually low. After thawing recovery ranged from 2 - 35%. These cells showed low Tac and variable APO-1 expression (3 - 15% and 1 - 53% positive cells, respectively). For further studies, the cells were cultured in medium supplemental with IL-2 for 5 days. Under these conditions ATL cells increased APO-1 and Tac expression. Again the intensity of APO-1 expression was comparable to the one on HTLV-1 transformed T cell lines and on other sensitive cells such as activated T cells or malignant T or B cell lines.

## Effect of anti-APO-1 on proliferation of ATL cells in vitro

Since APO-1 was expressed on cultured ATL cells we tested whether anti-APO-1 inhibited proliferation of these cells in vitro. ATL cells were cultured either in medium alone or in medium plus IL-2 and IL-4. The reason for culturing the cells in IL-2 or IL-4 was that in some cases ATL cells showed a proliferative response to IL-2 or IL-4 without prior activation. Arima N., Daitoku T., Ohgaki S. et al., "Autocrine growth of interleukin-2 producing cells in a patient with adult T cell leukemia", Blood 1986; 68: 779-782; Uchiyama T., Kamio M., Kodaka T. et al., "Leukemic cells from some adult T cell leukemia patients proliferate in response to interleukin-4", Blood 1988; 72: 1182-1186. Table 1 shows that ATL cells from patients 1 and 4 already exhibited a high spontaneous proliferation. In cells from patients 3, 4 and 5 proliferation was augmented by addition of IL-2 or IL-4. Addition of anti-APO-1 to the cultures greatly inhibited the proliferation of the malignant cells. Incubation with a control antibody did not have any effect.

Table 2

| Effect of Anti-APO-1 on proliferation of ATL cells in vitro | | | |
|---|---|---|---|
| cells from patient No. | incubation with | proliferation of cells in medium (cpm) | growth inhibition by anti-APO-1 (%) |
| 1 | medium<br>IL-2<br>IL-4 | 24682<br>18872<br>49343 | 82<br>73<br>61 |
| 2 | medium<br>IL-2<br>IL-4 | n.d.<br>110167<br>43674 | -<br>99<br>99 |
| 3 | medium<br>IL-2<br>IL-4 | 420<br>3700<br>5253 | 18<br>89<br>97 |
| 4 | medium<br>IL-2<br>IL-4 | 36672<br>46716<br>61503 | 48<br>52<br>60 |
| 5 | medium<br>IL-2<br>IL-4 | 629<br>1724<br>2110 | 10<br>71<br>81 |

After thawing $2 \times 10^5$ cells/well were cultured in a 96 well flat bottom plate. Where indicated IL-2 (20 U/ml) or IL-4 (5ng/ml) were added. The cells were cultured for 3 days in the presence of medium alone or with 1 $\mu$g/ml anti-APO-1 or 1 $\mu$g/ml isotype matched control antibody. 0.5 $\mu$Ci of H-TdR were added for the last 8 h of culture.

Proliferation was measured by determining H-TdR uptake. Data are given as the absolute cpm (mean) for culture in medium alone (cpm control) and the percentage of inhibition of H-TdR uptake by anti-APO-1. The standard deviation for triplicate cultures was less than 10%. Incubation with the isotype matched control antibody had no effect compared to culture in medium alone (not shown).

Induction of apoptosis of cultured ATL cells by anti-APO-1 treatment in vitro

To investigate the induction of apoptosis of ATL cells by anti-APO-1 thawed ATL cells were first cultured for five days in the presence of IL-2 (20 U/ml). During this time no net gain in cell numbers during culture was observed. The cultured cells were then incubated for 48 hours with 1 $\mu$g/ml anti-APO-1 or control antibody, respectively. Under these conditions 75 - 100% of ATL cells were dead after anti-APO-1 treatment.

Conclusion

All HTLV-I positive T cell lines, originally established from patients with ATL, were found to express APO-1. Incubation of these cells with anti-APO-1 led to inhibition of proliferation via induction of apoptosis similar to activated T cells. The sensitivity of HTLV-I positive T cell lines for anti-APO-1 mediated apoptosis varied between different cell lines (antibody concentration necessary for 50% of growth inhibition: 70 - 50 ng/ml ⟨JGCL and MJCL⟩ and 1000 - 2000 ng/ml ⟨HUT102 and C91/PI⟩). The most sensitive cell lines (JGCL and MJCL) were as sensitive as the most sensitive cell line (SKW6.4) found in previous experiments and against which the antibody was originally raised. Interestingly, proliferation of JGCL and MJCL appeared to depend on exogenous IL-2. This may suggest a connection between IL-2 responsiveness and sensitivity for anti-APO-1 induced apoptosis.

Expression of APO-1 was also found on cultured cells from the peripheral blood of patients with ATL. Thawed cells from such patients could not directly be studied since the viability was too low after thawing. However, when cultured in vitro in medium alone or in the presence of cytokines (IL-2 or IL-4) these cells were found to express APO-1. Treatment of these cells in vitro with anti-APO-1 strongly inhibited the spontaneous proliferation and the IL-2 or IL-4 augmented proliferative responses. Finally, the inhibition of proliferation was accompanied by the induction of apoptotic cell death. These data on cultured ATL are in contrast to the data obtained with normal T cells. In normal T cells APO-1 expression needs prior activation with mitogens and anti-APO-1 treatment has no effect on short term proliferation.

Taken together we have demonstrated that expression of APO-1 is a characteristic feature of HTLV-I transformed T cell lines and cultured malignant T cells from patients with ATL. Incubation of these cells and cell lines with the anti-APO-1 antibody induces apoptosis.

**Claims**

1. Use of an antibody which specifically binds to an antigen associated with cellular apoptosis under conditions appropriate for antibody mediated cellular apoptosis for the preparation of a therapeutic composition for the inhibition of the proliferation of a cell infected by the HTLV-1 virus.

2. The use according to claim 1 wherein the antigen is the APO-1 antigen.

3. The use of claim 2, wherein the antibody is a monoclonal antibody.

4. Use of an antibody which specifically binds to an antigen associated with cellular apoptosis under conditions appropriate for antibody mediated cellular apoptosis for the preparation of a therapeutic composition for killing a substantial percentage of a population of cells infected by the HTLV-1 virus.

5. The use according to claim 4, wherein the antigen is the APO-1 antigen.

6. The use according to claim 5, wherein the antibody is a monoclonal antibody.

12

EP 0 511 202 B1

**Patentansprüche**

1. Verwendung eines Antikörpers, der spezifisch an ein mit der zellulären Apoptose assoziiertes Antigen unter Bedingungen, die für die Antikörper vermittelte zelluläre Apoptose geeignet sind, bindet, zur Herstellung eines therapeutischen Präparats zur Hemmung der Proliferation einer durch das HTLV-1 Virus infizierten Zelle.

2. Verwendung nach Anspruch 1, dadurch **gekennzeichnet,** daß das Antigen das APO-1 Antigen ist.

3. Verwendung nach Anspruch 2, dadurch **gekennzeichnet,** daß der Antikörper ein monoklonaler Antikörper ist.

4. Verwendung eines Antikörpers, der spezifisch an ein mit der zellulären Apoptose assoziiertes Antigen unter Bedingungen, die für die Antikörper vermittelte zelluläre Apoptose geeignet sind, bindet, zur Herstellung eines therapeutischen Präparats zur Abtötung eines wesentlichen Prozentsatzes einer Population von durch das HTLV-1 Virus infizierten Zellen.

5. Verwendung nach Anspruch 4, dadurch **gekennzeichnet,** daß das Antigen das APO-1 Antigen ist.

6. Verwendung nach Anspruch 5, dadurch **gekennzeichnet,** daß der Antikörper ein monoklonaler Antikörper ist.

**Revendications**

1. Utilisation d'un anticorps qui se lie spécifiquement à un antigène associé à l'apoptose cellulaire dans des conditions appropriées pour une apoptose cellulaire médiatisée par l'anticorps pour la préparation d'une composition thérapeutique pour l'inhibition de la prolifération d'une cellule infectée par le virus HTLV-1.

2. Utilisation suivant le revendication 1, dans laquelle l'antigène est l'antigène APO-1.

3. Utilisation suivant le revendication 2, dans laquelle l'anticorps est un anticorps monoclonal.

4. Utilisation d'un anticorps qui se lie spécifiquement à un antigène associé à l'apoptose cellulaire dans des conditions appropriées pour une apoptose cellulaire médiatisée par l'anticorps pour la préparation d'une composition thérapeutique pour tuer une pourcentage substantiel d'une population de cellules infectées par le virus HTLV-1.

5. Utilisation suivant le revendication 4, dans laquelle l'antigène est l'antigène APO-1.

6. Utilisation suivant le revendication 5, dans laquelle l'anticorps est un anticorps monoclonal.

13

FIG. I

FIG. 2A

FIG. 2B

FIG. 2C

FIG. 4A

FIG. 4B

FIG. 3